(19) Europäisches Patentamt
European Patent Office
Office européen des brevets

(11) EP 1 232 008 B1

(12) **EUROPEAN PATENT SPECIFICATION**

(45) Date of publication and mention of the grant of the patent:
**23.07.2003 Bulletin 2003/30**

(21) Application number: **00974489.7**

(22) Date of filing: **27.10.2000**

(51) Int Cl.[7]: **B01J 31/40**, C07C 45/78, B01D 61/02, C07C 67/347

(86) International application number:
**PCT/EP00/10699**

(87) International publication number:
**WO 01/037993 (31.05.2001 Gazette 2001/22)**

# (54) PROCESS TO SEPARATE A RHODIUM/PHOSPHITE LIGAND COMPLEX AND FREE PHOSPHITE LIGAND COMPLEX FROM A HYDROFORMYLATION MIXTURE

VERFAHREN ZUR ABTRENNUNG EINES RHODIUMPHOSPHITLIGANDKOMPLEXES UND FREIEN PHOSPHITLIGANDES AUS HYDROFORMYLIERUNGSMISCHUNGEN

PROCEDE PERMETTANT DE SEPARER UN COMPLEXE DE LIGANDS DE RHODIUM/PHOSPHITE ET UN COMPLEXE DE LIGANDS DE PHOSPHITE LIBRE A PARTIR D'UN MELANGE D'HYDROFORMYLATION

(84) Designated Contracting States:
**AT BE CH CY DE DK ES FI FR GB GR IE IT LI LU MC NL PT SE**

(30) Priority: **23.11.1999 EP 99203927**

(43) Date of publication of application:
**21.08.2002 Bulletin 2002/34**

(73) Proprietor: **DSM N.V.**
**6411 TE Heerlen (NL)**

(72) Inventors:
- **GELLING, Onko, Jan**
  **NL-6129 HV Urmond (NL)**
- **BORMAN, Peter, Cornelis**
  **NL-6163 LE Geleen (NL)**
- **SMITS, Hubertus, Adrianus**
  **NL-6212 GC Maastricht (NL)**
- **CAUWENBERG, Veerle**
  **B-3010 Leuven (BE)**
- **VERGOSSEN, Franciscus, Hendrik, Paul**
  **NL-6104 AR Echt (NL)**

(74) Representative: **Verhaegen, Ilse Maria M.**
**DSM Patents & Trademarks,**
**Office Geleen,**
**P.O. Box 9**
**6160 MA Geleen (NL)**

(56) References cited:
**EP-A- 0 810 029**
**EP-A- 0 839 794**
**WO-A-96/34687**
**GB-A- 1 312 076**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 232 008 B1

Printed by Jouve, 75001 PARIS (FR)

## Description

The invention relates to a process to separate a rhodium/phosphite ligand complex and free phosphite ligand from a hydroformylation mixture also containing high boiling hydroformylation compounds by contacting said mixture with a polymeric membrane.

With hydroformylation is meant the reaction of an ethylenically unsaturated organic compound with hydrogen and carbon monoxide in the presence of a hydroformylation catalyst, in which an aldehyde compound is obtained.

Compounds having a higher boiling point than the aldehyde product are designated as high boiling hydroformylation compounds in this specification. Examples are aldehyde condensation by-products, for example dimers, trimers and tetramers of the aldehyde.

A problem in hydroformylation processes is the excessive accumulation of such high boiling compounds in the recirculating catalyst stream. The recirculating catalyst stream is the stream which originates after the recovery of the hydroformylation aldehyde product. It is therefore necessary to separate the high boiling compounds from the recirculating catalyst stream by means of a purge. The presence of a high boiler purge has been found to be a problem because such purge is a potential source of catalyst, i.e. rhodium, loss. Rhodium metal is extremely expensive and significant losses can easily make the operation of commercial plants uneconomic. For a commercially interesting process it is therefore necessary to recover the catalyst system comprising the rhodium/phosphite ligand complex and free phosphite ligand from such a high boiler hydroformylation purge stream.

A process for separating rhodium and phosphite ligand from a mixture also containing high boiling hydroformylation compounds is described in WO-A-9634687. In Example 3 of this publication rhodium and a bidentate phosphite ligand are separated from a mixture containing rhodium, a bidentate phosphite ligand, butyraldehyde and 10 wt.% trimer of butyraldehyde using a polydimethylsiloxane composite membrane obtained from Membrane Products Kiryat Weizmann. WO-A-9634687 does not exemplify the separation of rhodium and phosphite ligand from a high boiler hydroformylation purge.

It has been found that, when the membrane separation process as described in WO-A-9634687 is used for separating rhodium and phosphite ligand from a high boiler hydroformylation purge, the flux through the membrane is relatively small which prevents it from being a commercially attractive process.

The efficiency of a given membrane is determined by two parameters: its selectivity and the flow through the membrane. The latter, often denoted as the flux or permeation rate, is defined as the volume flowing through the membrane per unit area and time. The selectivity of a membrane towards a mixture is generally expressed by the retention R. The cut-off of a given membrane is defined as that molecular weight which is at least 90% rejected by the membrane. A membrane having a cut-off of x g/mole means that all compounds with a molecular weight greater than x are 90% or more rejected.

The object of the invention is to provide a membrane separation process for separating a rhodium/phosphite ligand complex and free phosphite ligand from a hydroformylation mixture also containing high boiling hydroformylation compounds with an improved flux.

This object is achieved in that said mixture is diluted with at least one diluent compound having a solubility parameter such that the absolute difference in relation to the solubility parameter of the polymeric membrane lies between 0 and 400 $\sqrt{kJ/m^3}$ and the ratio of the molar volume of the phosphite ligand and said diluent compound is ≥ 1.5, to such an extent that the amount of high boiling compounds in said mixture is ≤ 50 wt.% (relative to the total amount of the mixture) and the amount of compounds with a molecular weight of between x ± 200 is ≤ 20 wt.% (relative to the total amount of the mixture), whereby x is the cut-off of the membrane (g/mole).

It has been found that with the process according to the invention the flux of the membrane separation process is increased more than proportional to the dilution factor. An advantage of the process according to the invention is that a smaller volume of area of membrane process equipment can be used. From an economical/investment point of view smaller process equipment is desired.

Dilution of the mixture which is fed to the membrane with a solvent is a technique known in the art for washing out low molecular weight compounds. This technique is called diafiltration. Dilution of 1 part of the feed with 1 part of solvent suggests that a double amount of membrane area is required in order to permeate the doubled amount of feed. It has surprisingly been found that in the process according to the invention dilution of the feed with a factor x with at least one of the above mentioned diluent compounds advantageously results in an increase of the flux with a factor larger than x and hence results in a relative decrease of the membrane area required.

The diluent compounds used in the process according to the invention can be any compound having a solubility parameter such that the absolute difference in relation to the solubility parameter of the polymeric membrane lies between 0 and 400$\sqrt{kJ/m^3}$. The absolute difference between the solubility parameter of the diluent compound and the solubility parameter of the polymeric membrane is preferably between 0 and 300 $\sqrt{kJ/m^3}$ and more preferably between 0 and 200$\sqrt{kJ/m^3}$. The ratio of the molar volume of the phosphite ligand and said diluent compound is preferably ≥ 3, more preferably ≥ 5.

The solubility parameter of the diluent compound, the aldehyde compound and the high boiling compounds may be readily calculated according to the group contribution method described in AIChE J., 40(10), p. 1697-1710 (1994) "New Group Contribution Method for Estimating Properties of Pure Compounds", developed by L. Constantinou and R. Gani and Fluid Phase Equilibria, 103(1), p. 11-22 (1995), "Estimation of the Acentric Factor and the Liquid Molar Volume at 298K through a new group Contribution Method", developed by L. Constantinou, R. Gani, and J.P. O'Connell.

The solubility parameter of the polymeric membrane can be obtained from literature sources. Illustrative solubility parameters of various polymer membranes are given in WO-A-9634687.

The molar volume of the phosphite ligand and diluent compound may be calculated according to the above mentioned group contribution method described in Fluid Phase Equilibria, 103 (1), p. 11-22 (1995).

The polymeric membrane used in the process of the invention is preferably supported on a porous support. Such support helps in providing mechanical strength while the actual desired separation of the process of the invention is provided by the particular polymeric membrane employed. Examples of porous support materials are mentioned in WO-A-9634687 which is herein incorporated as reference. A polymeric membrane supported on a porous support is designated as composite membrane in this specification.

By free ligand is meant organophosphite ligand that is not complexed with (tied to or bound to) the rhodium atom of the complex catalyst. Examples of suitable phosphite ligands are given in WO-A-9634687 and WO-A-9733854 which are herein incorporated as references.

In the process according to the invention the concentration of the high boiling compounds in the mixture which is fed to the membrane may not exceed 50 wt.%. Preferably the concentration of the high boiling compounds is ≤ 30 wt.%, more preferably ≤ 25 wt.%.

The sum of the amounts of high boiling compounds, ethylenically unsaturated organic starting compound(s), aldehyde compound(s), diluent compound(s), rhodium/phosphite ligand complex, degradation products thereof and free phosphite ligand is 100 wt.%.

The concentration of the rhodium complex in the liquid to be treated is not critical. In a hydroformylation high boiler purge, the concentration will generally be higher than 25 ppm rhodium and lower than 3000 ppm rhodium. Preferably, the rhodium concentration is higher than 100 ppm and lower than 1200 ppm. More preferably, the rhodium concentration is equal to or higher than 200 ppm and equal to or lower than 800 ppm.

The high boiler hydroformylation mixture containing the hydroformylation catalyst and high boiling compounds will generally comprise also 25-80 wt.% of the aldehyde product and 0.5-5 wt.% ligand degradation products.

In the process according to the invention, a substantial portion of the high boiling compounds, diluent compound(s) and - if present - hydroformylation reaction product is passed through the membrane while retaining a substantial portion of the rhodium/phosphite ligand complex catalyst and free phosphite ligand. Thereafter high boiling compounds, diluent compound(s) and - if present - hydroformylation reaction product which passes through the membrane as permeate is removed for further downstream treatment. The rhodium/phosphite ligand complex catalyst and free phosphite ligand is retained as a retentate and is recycled to the hydroformylation reactor.

The diluent compound(s) and the hydroformylation reaction product - if present - in the permeate are separated in one or more separation steps according to conventional techniques. Advantageously the separation is performed using vacuum distillation.

The membrane used in the process according to the invention is chosen so that on bringing the mixture into contact with one side of the membrane at an applied pressure greater than the pressure on the opposite side of the membrane, the pressure differential being greater than the osmotic pressure of the system, the permeate has a reduced content of the rhodium/phosphite ligand complex. Suitable membranes include membranes which are stable in organic media, for example the polymeric composite membranes as for example described in WO-A-9634687 which is herein incorporated as reference. Preferred polymeric composite membranes are polydimethylsiloxane membranes as for example described in US-A-5265734 which is herein incorporated as reference.

The pressure of the feed stream of the membrane separation process according to the invention is generally between 0.1 and 4 MPa, preferably between 2 and 4 MPa. The process according to the present invention is performed using nano- or microfiltration. The pressure of the permeate is in general between 0.1 and 1 MPa, preferably between 0.1 and 0.4 MPa.

The temperature of the polymeric membrane separation process according to the invention depends on the type of polymer used and is restricted by the temperature stability of the polymeric membrane. The temperature is generally between 10 and 50°C and preferably between 25 and 40°C.

The high boiler purge is generally removed continuously or intermittently from the hydroformylation system. The purge may be removed either directly from the reactor or, for example, from some point in the catalyst recycle stream.

The claimed process can be performed batchwise or continuously, in one or more stages.

With the one-step variant, the feed solution is added to the membrane under pressure. In the simplest case the permeate is drawn off and the concentrated solution is removed from the separating device as soon as the desired

concentration has been reached. This procedure can also be performed continuously to increase the separating efficiency. The feed solution then flows along the membrane, is concentrated and continuously drawn off, as is the permeate.

Multi-stage separation is performed with separating stages either in parallel and/or in series. The series configuration, in which the concentrate is separated in every stage and the permeate solution is passed to the next separation stage, is especially advantageous for increasing the recovery of the rhodium/phosphite ligand complex and free phosphite.

The high boiler purge is preferably removed from a hydroformylation system for the preparation of a linear (or terminal) aldehyde obtainable by hydroformylation of unsaturated olefins substituted with one or more functional groups according to formula (1) or (2):

$$CH_3\text{-}CR^9\text{= }CR^{10}\text{-}R^{11} \qquad (1)$$

$$CH_3\text{= }CR^9\text{-}CR^{10}\text{-}R^{11} \qquad (2)$$

where $R^9$ and $R^{10}$ are a hydrocarbon group or preferably hydrogen and $R^{11}$ is a cyanide group or a hydrocarbon group, whether or not substituted with one or more functional groups which contain a hetero atom, for example oxygen, sulphur, nitrogen or phosphorus. Preferably, use is made of unsaturated olefins having between 4 and 20 carbon atoms according to formula (1) and/or (2), where $R^9$ and $R^{10}$ are hydrogen.

Examples of unsaturated olefins having between 4 and 20 carbon atoms according to formula (1) and (2), where $R^9$ and $R^{10}$ are hydrogen, are 2-pentene nitrile, 3-pentene nitrile, 4-pentene nitrile, 4-pentenoic acid, 3-pentenoic acid and $C_1$-$C_6$-alkyl ester of 4-pentenoic acid and 3-pentenoic acid. The resulting aldehyde hydroformylation products, in particular methyl-5-formylvalerate, are intermediate products in the preparation of ε-caprolactam or adipic acid, which are in turn raw materials for the preparation of nylon-6 and nylon-6,6, respectively. Examples of $C_1$-$C_6$-alkyl-3-pentenoate esters and $C_1$-$C_6$-alkyl-4-pentenoate esters are methyl-, ethyl-, propyl-, isopropyl-, tert-butyl-, pentyl-and cyclohexyl-3-pentenoate and 4-pentenoate. Preferably, use is made of methyl-3-pentenoate, and/or methyl-4-pentenoate or of ethyl-4-pentenoate and/or ethyl-3-pentenoate because these compounds are readily obtainable. 3-pentene nitrile, 3-pentenoic acid and $C_1$-$C_6$-alkylesters of pentenoic acid may be present in the reaction mixture as a mixture which also comprises 2- and 4-pentenenitrile, 2-and 4-pentenoic acid and $C_1$-$C_6$-alkylesters of 2- and 4-pentenoic acid, respectively.

The process according to the invention will be described in more detail for the following preferred embodiments. It shall be evident that the below stated conditions will also be applicable for the above described starting mixtures in a manner clear to one skilled in the art.

A preferred embodiment is when the hydroformylation mixture which is treated in the process to the invention is derived from a hydroformylation process for the preparation of an alkyl 5-formylvalerate by reacting alkyl 3-pentenoate and/or alkyl 4-pentenoate with carbon monoxide and hydrogen using a rhodium/bidentate phosphite ligand complex catalyst as for example described in WO-A-9733854 which is herein incorporated as reference.

Examples of suitable diluent compounds in this preferred embodiment of the invention are the alkyl 5-formylvalerate compounds and its isomers, alkyl 4-pentenoate and its isomers and alkyl valerate and mixtures containing at least two of these compounds. Preferred diluent compounds are alkyl valerate compounds, alkyl pentenoate compounds, preferably alkyl-3-pentenoate, or a mixture containing alkyl valerate and one or more alkyl pentenoate compound(s). It has been found that in this preferred embodiment dilution of the high boiler purge with alkyl valerate, alkyl pentenoate or with a mixture containing alkyl valerate and one or more alkyl pentenoate compounds is especially advantageous because this results in a further decrease of required membrane area. Another advantage of using alkyl valerate and/or alkyl pentenoate is that the retentate of the membrane separation process, containing rhodium/phosphite ligand complex, free phosphite ligand and diluent compound(s) can be recycled to the hydroformylation reactor without further treatment. This is because alkyl pentenoate is the starting product to be hydroformylated and alkyl valerate is formed as a by-product in the hydroformylation of alkyl-3-pentenoate and/or alkyl-4-pentenoate.

Alkyl valerate is a compound which can for example be formed through hydrogenation of the corresponding pentenoate compound. For example, methyl valerate is formed as a by-product when hydroformylating methyl-3-pentenoate and/or methyl-4-pentenoate into methyl-5-formylvalerate.

The process according to the invention is advantageously to be used in a process for the continuous preparation of an alkyl 5-formylvalerate by hydroformylating alkyl-3- and/or 4-pentenoate in the presence of a hydroformylation catalyst. First the alkyl 5-formylvalerate is separated from the rhodium/phosphite ligand complex catalyst in a conventional separation unit, for example using vacuum distillation, resulting in a fraction containing the alkyl 5-formylvalerate and a fraction containing the catalyst system and high boiling compounds. Subsequently a part of the organic mixture containing the catalyst system and high boiling compounds is purged and the rhodium/phosphite ligand complex and free phosphite ligand are separated from the high boiling compounds with the process according to the invention and subsequently recycled to the reactor.

In this preferred embodiment of the invention the greatest part of the high boiling compounds has a molecular weight $M_w$ of between 200 and 1000. A typically distribution of the high boiling compounds is that 70% of all the high boiling compounds has a molecular weight less than 600, 25% has a molecular weight of between 600 and 1000 and 5% has a molecular weight more than 1000.

The molar ratio of the multidentate phosphite ligand to rhodium in the hydroformylation reaction mixture and the high boiler purge to be treated is generally from 0.5 to 100 and preferably from 1 to 10 and most preferably less than 1.2 (mol ligand/mol metal). Preferably the ratio is higher than 1.05. It has been found that in this range the ligand stability during hydroformylation is optimal and the loss of ligand in the high boiler purge according to this invention is minimal.

The invention is also directed to a process to prepare alkyl 5-formylvalerate, wherein the following steps are performed:

(a) hydroformylation of an alkyl-3- and/or 4-pentenoate in the presence of a catalyst system containing rhodium and a multidentate organic phosphite catalyst, whereby the hydroformylation reaction mixture contains 1-10, preferably 1.05-1.2 mol multidentate phosphite ligand per mol rhodium,
(b) separating the catalyst system from the reaction mixture obtained in step (a) resulting in a fraction containing the linear and branched aldehyde compounds, unconverted starting compound and double-bond isomers thereof and a fraction containing the catalyst system and high boiling compounds,
(c) purging part of the fraction containing the catalyst system and high boiling compounds and recycling the rest to step (a),
(d) separating the catalyst system from the purge with the process according to the invention,
(e) reuse of the catalyst system obtained in step (d) in step (a),
(f) separating the fraction containing linear and branched aldehyde compounds, unconverted starting compound and double-band isomers thereof obtained in step (b) in two or more separation steps in a fraction containing the linear aldehyde, a fraction containing the branched aldehyde compounds, a fraction containing the unconverted starting compound and double-bond isomers thereof and a fraction containing at least alkyl valerate compound,
(g) recycling the fraction containing the unconverted starting compound and double-bond isomers thereof obtained in step (f) to step (a),
(h) feeding at least a part of the fraction containing at least alkyl valerate compound to step (d).

The above described preferences regarding hydroformylation starting compound, conditions and catalyst system also apply here. Step (a) is preferably performed as described in for example US-A-5527950, EP-A-712828 or WO-A-9518089. Step (b) may be performed using any separation technique known to a person skilled in the art. Examples of suitable separation techniques are (vacuum) distillation, crystallisation, extraction using a suitable extraction agent and membrane separation as for example described in WO-A-9634687.

A possible process according to the invention is schematically represented in Figure 1. Figure 1 will be elucidated in a non-limitative manner below to illustrate the preparation of methyl-5-formylvalerate.

In Figure 1, methyl-3-pentenoate and/or methyl-4-pentenoate is fed to reactor (A) via stream (1). In Reactor A the catalyst system is present. A mixture of CO and $H_2$ is fed to the reactor (A) via stream (2). The effluent of reactor (A) comprising methyl-5-formylvalerate, methyl-3-formylvalerate, methyl-4-formylvalerate, methyl-2-formylvalerate, low-boiling by-products (for example methyl valerate, methyl-2-pentenoate and methyl 4-pentenoate), high boiling compounds, any unconverted methyl-3-pentenoate, the catalyst system, carbon monoxide and hydrogen is fed to flasher (B) via stream (3). In the flasher (B) the pressure is reduced to for example atmospheric pressure. Carbon monoxide and hydrogen are separated from the reaction mixture via stream (4) and recycled to the reactor (A). The resulting liquid mixture is fed to ion exchanger (C), a packed bed of a polystyrene matrix containing basic amine groups, via stream (5). The effluent is fed to separation step (D) via resulting liquid stream (6). In separation step (D) the catalyst system and high boiling compounds and some methyl-5-formylvalerate is separated from the liquid mixture (stream 7b). A part of stream (7b) is purged (stream (8)) and the rest is recycled to the reactor (A). The purge stream (8) is treated according to the process of present invention in the membrane separation unit (E) containing one or more polymeric membranes in series and/or in parallel. The purge stream is somewhere in the membrane separation unit (E) diluted with at least one of the above mentioned diluent compounds. In the membrane separation unit (E) the diluted high-boiler purge is split into a concentrate stream (8a) and a permeate stream (8b). The concentrate containing catalyst system and free phosphite ligand is recycled to the reactor (A). The permeate stream (8b) containing methyl-5-formylvalerate, methyl valerate and high boiling compounds is fed to separation step (F), preferably a vacuum distillation

unit. In case methyl valerate is used as diluent compound, the liquid mixture is split in separation step (F) into methyl valerate (stream 9a) and methyl-5-formylvalerate and high boiling compounds (stream 9b). The mixture containing methyl valerate (stream 9a) is optionally for at least a part fed to separation step (E). Stream (9b) is fed to separation step (G) in which methyl 5-formylvalerate (stream 10a) is separated from the high boiling compounds (stream 10b). Stream (7a), leaving the separation unit (D) contains the most of the volatile components, for example methyl-2-pentenoate, methyl-4-pentenoate, methylvalerate, most of the unconverted methyl-3-pentenoate and the most of the aldehyde products. Stream (7a) is fed to separation step H, preferably a vacuum distillation unit. In separation step H methyl-5-formylvalerate and its branched isomers are discharged via stream (11 b). Stream (11b) is fed to separation step J, preferably a vacuum distillation unit. In separation step J the branched methyl formyl valerates are discharged via stream (12a) and methyl-5-formyl valerate is discharged via stream (12b). Stream (11a) is fed to separation step (I), preferably a distillation unit. In separation step (I) methylvalerate, methyl-4-pentenoate and cis-methyl-2-pentenoate are discharged via stream (13a). Stream (13a) is at least partially fed to separation step (E). Optionally the mixture of stream (13a) is treated with a hydrogenation catalyst in order to hydrogenate the methyl pentenoate compounds to methylvalerate. Trans-methyl-2-pentenoate and methyl-3-pentenoate are recirculated to reactor (A) via stream (13b).

The present invention also relates to a process for the separation of rhodium/phosphite ligand complex and free phosphite ligand from a hydroformylation reaction mixture, also containing aldehyde hydroformylation reaction product(s), ethylenically unsaturated starting compounds and optionally high boiling hydroformylation compounds in which the hydroformylation reaction mixture is contacted with an inorganic membrane. Suitable inorganic membranes are for example ceramic membranes formed form alumina, silica or zirconia. The use of inorganic membranes is advantageous because they exhibit superior thermal stability relative to polymeric membranes. The inorganic membrane separation process can therefore be operated at higher temperatures resulting in an improved flux through the membrane.

The invention will be elucidated by the following examples, however these are not intended to limit the scope of the invention in any way.

Comparative Experiment A

This experiment demonstrates that a low flux is obtained when the concentration of high boilers in the mixture is approximately 51 wt.%

In a continuous process according to figure 1, the hydroformylation substrate methyl-3-pentenoate is reacted with CO and $H_2$ to form a mixture of methyl formyl valerates. This is done by contacting methyl-3-pentenoate with a catalyst comprising Rh, a bidentate phosphite ligand ($M_w$ = 1090) with formula (3)

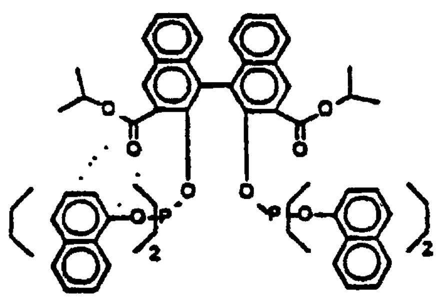

(molar ratio ligand/rhodium = 1.05-1.2 mol/mol) and a mixture of CO and $H_2$ (95°C, CO:H2 = 1:1 mol/mol, P = 0.5 MPa, residence time = 6 hrs total in three well-mixed reactors in series). After partial separation of the products from the reaction mixture by vacuum evaporation, the stream containing the non-volatile catalyst is recycled continuously to the reactor section.

In this process the selectivity to high boilers is 0.2-1.5%. In this experiment the high boiler compounds which are formed are allowed to accumulate in the recirculating catalyst stream until their concentration is approximately 51 wt% (the remainder of the mixture comprises the catalyst system, degradation products thereof, reaction products and the hydroformylation substrate). The recirculating catalyst stream typically contains 360 ppm Rh (before dilution with a diluent compound). When the concentration of high boilers in the recirculating catalyst stream has reached 51 wt%, a desired amount of material is purged from the recirculating catalyst stream and is passed over a membrane MPF-50® (a polydimethylsiloxane composite membrane obtained from Membrane Products Kiryat Weizmann with solubility parameter $471\sqrt{kJ/m^3}$ and a cut-off of 700 g/mole) of in an off-line membrane separation unit according to figure 2.

The pressure on the retentate side is 0.3 MPa, the pressure on permeate side is approximately 0.1 MPa. The operating temperature is 27°C. The initial flux through the membrane is 0.74 kg/($m^2$/hr) only and drops to lower values as the experiment is progressing.

EXAMPLE 1

Experiment A is repeated except that the high boiler hydroformylation purge mixture is diluted with various amounts of methylvalerate before contacting the purge mixture with the membrane. The solubility parameter of methylvalerate is 573 $\sqrt{kJ/m^3}$. The difference in solubility parameter between the membrane and methylvalerate is 102 $\sqrt{kJ/m^3}$. The molar volume of methylvalerate is 0.121 $m^3$/kmol and the molar volume of the phosphite ligand with formula (3) is 0.932 $m^3$/kmol. The ratio of the molar volume of the phosphite ligand and methylvalerate is 7.7.
Table 1 shows that dilution of the high boiler hydroformylation purge mixture with methylvalerate results in a flux improvement which is much larger than the dilution factor. The distribution of the high boilers is as follows:

70% of the high boilers has a molecular weight < 630,
25% of the high boilers has a molecular weight of between 630 and 970 and
5% has a molecular weight of more than 970.

Table 1:

| Flux improvement by dilution with methylvalerate | | | |
|---|---|---|---|
| concentration high boilers [wt%] after dilution | flux [kg/m2/hr] | flux improvement factor (1) | dilution factor (2) |
| 22.4 | 9.2 | 12.5 | 2.2 |
| 24.2 | 6.0 | 8.1 | 2.1 |
| 25.5 | 8.1 | 10.9 | 2.0 |
| 27.4 | 6.6 | 8.9 | 1.8 |
| 29.3 | 5.0 | 6.8 | 1.7 |
| 29.4 | 4.7 | 6.4 | 1.7 |
| 30.4 | 5.4 | 7.3 | 1.6 |

(1) flux improvement factor = flux after dilution/flux before dilution
(2) dilution factor = concentration of high boiling compounds in the mixture before dilution/concentration of high boiling compounds in the mixture after dilution.

EXAMPLE 2

This example demonstrates that the separation process according to the invention can be carried out with good catalyst recovery and adequate high boiler removal in a very wide concentration factor(2) range.
Experiment A is repeated except that the high boiler hydroformylation purge mixture is diluted with methylvalerate before contacting the purge mixture with the membrane such that the mixture contains 25 wt.% of high boilers. Samples are taken on the retentate (concentrate) and permeate side of the membrane at three different concentration factors(2). The Rh retention is given in Table 2.

Table 2:

| Rh retention vs. concentration factor | | | |
|---|---|---|---|
| [Rh] in concentrate [ppm] | [Rh] in permeate [ppm] | Rh retention [%](1) | Conc. factor [-] (2) |
| 171 | 13 | 92.4 | 1.03 |
| 829 | 55 | 93.4 | 4.79 |

(1) Rh-retention = (1 - [Rh] permeate/[Rh] concentrate) *100%
(2) Concentration factor = Cf = initial mass of high boiler containing mixture /mass of concentrate

Table 2: (continued)

| Rh retention vs. concentration factor | | | |
|---|---|---|---|
| [Rh] in concentrate [ppm] | [Rh] in permeate [ppm] | Rh retention [%](1) | Conc. factor [-] (2) |
| 1255 | 95 | 92.4 | 13.0 |

(1) Rh-retention = (1 - [Rh] permeate/[Rh] concentrate) *100%

(2) Concentration factor = Cf = initial mass of high boiler containing mixture /mass of concentrate

Clearly, the retention is higher than 90% and constant for a very wide concentration factor range. The example also shows that by using a multistage membrane separation process, very high Rh-recovery (>99 %)can be obtained.

The retention of high boilers, catalyst complex and free ligand depends on the molar weight. The cut-off of the membrane used in these examples is 700 g/mole. This means that high boilers such as dimers and trimers of methyl-5-formylvalerate which have a molar weight << 700 g/mole will tend to permeate through the membrane, whereas very heavy high boilers and the catalyst complex and free ligand (molar weight >> 700 g/mole) will accumulate in the concentrate. This is illustrated by Table 3 below.

Table 3:

| Ratio's of concentrations as a function of molar weight for various concentration factors | | | |
|---|---|---|---|
| Average Mw [g/mole] | Cf = 1.03 C(conc)/ C(perm) | Cf = 4.79 C(conc)/ C(perm) | Cf = 13.0 C(conc)/ C(perm) |
| 1296 | 5.00 | 10.50 | 14.67 |
| 1092 | 3.75 | 3.86 | 4.18 |
| 802 | 5.00 | 4.96 | 5.05 |
| 587 | 2.71 | 3.08 | 2.50 |
| 479 | 2.08 | 1.75 | 1.55 |
| 369 | 1.53 | 1.53 | 1.61 |
| 369 | 1.05 | 1.16 | 1.15 |
| 143 | 0.90 | 0.92 | 1.00 |
| 101 | 0.80 | 0.90 | 0.85 |

From Table 3 it is very clear that species with a low molar weight (<< 700 g/mole) pass effectively through the membrane. It also shows that high molar weight species such as the catalyst complex and ligand (>> 700 g/mole) are well retained by the membrane. Thus, the process is very effective in separating catalyst from high boilers with a molar weight << 700 g/mole.

## Claims

1. Process to separate a rhodium/phosphite ligand complex and free phosphite ligand from a hydroformylation mixture also containing high boiling hydroformylation compounds,having a higher boiling point than the aldehyde product, by contacting said mixture with a polymeric membrane, wherein said mixture is diluted with at least one diluent compound having a solubility parameter such that the absolute difference in relation to the solubiltity parameter of the polymeric membrane lies between 0 and $400\sqrt{kJ/m^3}$ and the ratio of the molar volume of the phosphite ligand and said diluent compound is ≥ 1.5, to such an extent that the amount of high boiling compounds in said mixture is ≤ 50 wt.% (relative to the total amount of the mixture) and the amount of compounds with a molecular weight of between x ± 200 is ≤ 20 wt.% (relative to the total amount of the mixture) whereby x is the cut-off of the membrane.

2. A process according to claim 1, wherein the concentration of high boiling compounds is ≤ 30 wt.%.

**3.** A process according to claim 2, wherein the concentration of high boiling compounds is ≤ 25 wt.%.

**4.** A process according to any one of claims 1-3, wherein the absolute difference between the solubility parameter of the diluent compound and the solubiltity parameter of the membrane is between 0 and 200 $\sqrt{kJ/m^3}$.

**5.** A process according to any one of claims 1-4, wherein the ratio of the molar volume of the phosphite ligand and said diluent compound is ≥ 5.

**6.** A process according to any one of claims 1-5, wherein the hydroformylation mixture is derived from a hydroformylation process for the preparation of an alkyl 5-formylvalerate by reacting alkyl 3-pentenoate and/or alkyl 4-pentenoate with carbon monoxide and hydrogen using a rhodium/bidentate phosphite ligand complex catalyst.

**7.** Process according to claim 6, wherein the diluent compound is alkyl valerate or a mixture containing alkyl valerate and one or more alkyl pentenoate compounds.

**8.** Process for the preparation of an alkyl-5-formylvalerate, wherein the following steps are performed:

(a) hydroformylation of an alkyl-3- and/or 4-pentenoate in the presence of a catalyst system containing rhodium and a multidentate organic phosphite catalyst, whereby the hydroformylation reaction mixture contains 1.05-1.2 mol multidentate phosphite ligand per mol rhodium,
(b) separating the catalyst system from the reaction mixture obtained in step (a) resulting in a fraction containing the linear and branched aldehyde compounds, unconverted starting compound and double-band isomers thereof and a fraction containing the catalyst system and high boiling compounds,
(c) purging part of the fraction containing the catalyst system and high boiling compounds and recycling the rest to step (a),
(d) separating the catalyst system from the purge with the process according to any one of claims 1-7,
(e) reuse of the catalyst system obtained in step (d) in step (a),
(f) separating the fraction containing linear and branched aldehyde compounds, unconverted starting compound and double-band isomers thereof obtained in step (b) in two or more separation steps in a fraction containing the linear aldehyde, a fraction containing the branched aldehyde compounds, a fraction containing the unconverted starting compound and double-band isomers thereof and a fraction containing at least alkyl valerate compound,
(g) recycling the fraction containing the unconverted starting compound and double-band isomers thereof obtained in step (f) to step (a),
(h) feeding at least a part of the fraction containing at least alkyl valerate compound to step (d).

**9.** A process according to claim 1 to separate a rhodium/bidentate phosphite ligand complex and free bidentate phosphite ligand from a hydroformylation mixture also containing high boiling hydroformylation compounds by contacting said mixture with a polymeric membrane, wherein said hydroformylation mixture is derived from a hydroformylation process for the preparation of an alkyl 5-formylvalerate by reacting alkyl 3-pentenoate and/or alkyl 4-pentenoate with carbon monoxide and hydrogen using a rhodium/bidentate phosphite ligand complex catalyst and said mixture is diluted with alkyl valerate or a mixture containing alkyl valerate and one or more alkyl pentenoate compounds to such an extent that the amount of high boiling compounds in said mixture is ≤ 50 wt.% (relative to the total amount of the mixture) and the amount of compounds with a molecular weight of between x ± 200 is ≤ 20 wt.% (relative to the total amount of the mixture) whereby x is the cut-off of the membrane .

## Patentansprüche

**1.** Verfahren zur Abtrennung eines Rhodium/Phosphitligandenkomplexes und freiem Phosphitliganden von einem Hydroformylierungsgemisch, das auch hochsiedende Hydroformylierungsverbindungen enthält, die einen höheren Siedepunkt als die Aldehydprodukte aufweisen, durch das Kontaktieren des Gemisches mit einer polymeren Membran, wobei das Gemisch mit mindestens einer Verdünnungsmittelverbindung, welche einen solchen Löslichkeitsparameter aufweist, daß die absolute Differenz in Beziehung zu dem Löslichkeitsparameter der polymeren Membran zwischen 0 und 400 $\sqrt{kJ/m^3}$ liegt und das Verhältnis des Molvolumens des Phosphitliganden und der Verdünnungsmittelverbindung ≥ 1,5 ist, zu einem solchem Ausmaß verdünnt wird, daß die Menge an hochsiedenden Verbindungen in dem Gemisch ≤ 50 Gew.-% (relative zu der Gesamtmenge des Gemisches) ist und die Menge an Verbindungen mit einem Molekulargewicht zwischen x ± 200 ≤ 20 Gew.-% (relativ zu der Gesamtmenge des

Gemisches) ist, wobei x die Trenngrenze der Membran ist.

2. Verfahren nach Anspruch 1, wobei die Konzentration von hochsiedenden Verbindungen ≤ 30 Gew.-% ist.

3. Verfahren nach Anspruch 2, wobei die Konzentration von hochsiedenden Verbindungen ≤ 25 Gew.-% ist.

4. Verfahren nach einem der Ansprüche 1 bis 3, wobei die absolute Differenz zwischen dem Löslichkeitsparameter der Verdünnungsmittelverbindung und dem Löslichkeitsparameter der Membran zwischen 0 und 200 $\sqrt{kJ/m^3}$ ist.

5. Verfahren nach einem der Ansprüche 1 bis 4, wobei das Verhältnis des Molvolumens des Phosphitliganden und der Verdünnungsmittelverbindung ≥ 5 ist.

6. Verfahren nach einem der Ansprüche 1 bis 5, wobei das Hydroformylierungsgemisch von einem Hydroformylierungsverfahren zur Herstellung eines Alkyl-5-formylvalerats durch Umsetzen von Alkyl-3-pentenoat und/oder Alkyl-4-pentenoat mit Kohlenmonoxid und Wasserstoff unter Verwendung eines Katalysatorkomplexes von Rhodium/zweizähniger Phosphitligand herrührt.

7. Verfahren nach Anspruch 6, wobei die Verdünnungsmittelverbindung Alkylvalerat oder ein Gemisch, das Alkylvalerat und eine oder mehrere Alkylpentenoatverbindungen enthält, ist.

8. Verfahren für die Herstellung eines Alkyl-5-formylvalerats, worin die folgenden Schritte durchgeführt werden:

(a) Hydroformylierung eines Alkyl-3- und/oder 4-pentenoats in der Gegenwart eines Katalysatorsystems, das Rhodium und einen mehrzähnigen organischen Phosphitkatalysator enthält, wobei das Hydroformylierungsreaktionsgemisch 1,05 bis 1,2 mol mehrzähnigen Phosphitliganden pro Mol Rhodium enthält,
(b) Abtrennen des Katalysatorsystems von dem in Schritt (a) erhaltenen Reaktionsgemisch, was in einer Fraktion, welche die linearen und verzweigten Aldehydverbindungen, nicht-umgewandelte Ausgangsverbindung und Doppelbindungsisomere davon enthält, und in einer Fraktion, welche das Katalysatorsystem und hochsiedende Verbindungen enthält, resultiert,
(c) Ablassen eines Teils der Fraktion, die das Katalysatorsystem und hochsiedende Verbindungen enthält, und Rückführen des Rests in Schritt (a),
(d) Abtrennen des Katalysatorsystems von dem Abgelassenen mit dem Verfahren gemäß einem der Ansprüche 1 bis 7,
(e) Wiederverwenden des in Schritt (d) erhaltenen Katalysatorsystems in Schritt (a),
(f) Abtrennen der in Schritt (b) erhaltenen Fraktion, die lineare und verzweigte Aldehydverbindungen, nicht-umgewandelte Ausgangsverbindungen und Doppelbindungsisomere enthält, in zwei oder mehreren Abtrennungsschritten in eine Fraktion, welche den linearen Aldehyd enthält, eine Fraktion, welche die verzweigten Aldehydverbindungen enthält, eine Fraktion, welche die nicht-umgewandelte Ausgangsverbindung und Doppelbindungsisomere davon enthält, und eine Fraktion, welche mindestens Alkylvaleratverbindung enthält,
(g) Rückführen der in Schritt (f) erhaltenen Fraktion, welche die nicht-umgewandelte Ausgangsverbindung und Doppelbindungsisomere davon enthält, in Schritt (a),
(h) Beschicken mindestens eines Teils der Fraktion, welche mindestens Alkylvaleratverbindung enthält, in Schritt (d).

9. Verfahren nach Anspruch 1 zur Abtrennung eines Rhodium/zweizähniger Phosphitligandenkomplexes und freiem zweizähnigen Phosphitliganden von einem Hydroformylierungsgemisch, das auch hochsiedende Hydroformylierungsverbindungen enthält, durch durch das Kontaktieren des Gemisches mit einer polymeren Membran, wobei das Hydroformylierungsgemisch von einem Hydroformylierungsverfahren zur Herstellung eines Alkyl-5-formylvalerats durch Umsetzen von Alkyl-3-pentenoat und/oder Alkyl-4-pentenoat mit Kohlenmonoxid und Wasserstoff unter Verwendung eines Katalysatorkomplexes von Rhodium/zweizähniger Phosphitligand herrührt und das Gemisch mit einem Alkylvalerat oder einem Gemisch, welches Alkylvalerat und eine oder mehr Alkylpentenoatverbindungen enthält, zu einem solchem Ausmaß verdünnt wird, daß die Menge an hochsiedenden Verbindungen in dem Gemisch ≤ 50 Gew.-% (relative zu der Gesamtmenge des Gemisches) ist und die Menge an Verbindungen mit einem Molekulargewicht zwischen x ± 200 ≤ 20 Gew.-% (relativ zu der Gesamtmenge des Gemisches) ist, wobei x die Trenngrenze der Membran ist.

## Revendications

1. Procédé pour séparer un complexe de ligand rhodium/phosphite et un ligand phosphite libre à partir d'un mélange d'hydroformylation contenant aussi des composés d'hydroformylation à haut point d'ébullition, ayant un point d'ébullition plus élevé que les produits aldéhydiques, en mettant en contact ledit mélange avec une membrane polymérique, dans lequel ledit mélange est dilué avec au moins un composé diluant ayant un paramètre de solubilité tel que la différence absolue par rapport au paramètre de solubilité de la membrane polymérique, se situe entre 0 et $400\sqrt{kJ/m^3}$, et le rapport du volume molaire du ligand phosphite et dudit composé diluant est ≥ 1,5, jusqu'à que ce que la quantité des composés à haut point d'ébullition dans ledit mélange soit ≤ 50 % du poids (par rapport à la quantité totale du mélange), et la quantité des composés avec un poids moléculaire compris entre x ± 200 est ≤ 20 % du poids (par rapport à la quantité totale du mélange), par quel moyen x est la limite de la membrane.

2. Procédé selon la revendication 1, dans lequel la concentration en composés à haut point d'ébullition est ≤ 30 % du poids.

3. Procédé selon la revendication 2, dans lequel la concentration en composés à haut point d'ébullition est ≤ 25 % du poids.

4. Procédé selon l'une quelconque des revendications 1 à 3, dans lequel la différence absolue entre le paramètre de solubilité du composé diluant et le paramètre de solubilité de la membrane, se situe entre 0 et $200\sqrt{kJ/m^3}$.

5. Procédé selon l'une quelconque des revendications 1 à 4, dans lequel le rapport du volume molaire du ligand phosphite et dudit composé diluant est ≥ 5.

6. Procédé selon l'une quelconque des revendications 1 à 5, dans lequel le mélange d'hydroformylation est dérivé d'un procédé d'hydroformylation pour la préparation d'un 5-formylvalérate d'alkyle, en faisant réagir le 3-penténoate d'alkyle et/ou le 4-penténoate d'alkyle avec du monoxyde de carbone et de l'hydrogène, en utilisant un catalyseur au complexe de ligand rhodium/phosphite bidenté.

7. Procédé selon la revendication 6, dans lequel le composé diluant est un valérate d'alkyle ou un mélange contenant un valérate d'alkyle et un ou plusieurs composés penténoate d'alkyle.

8. Procédé pour préparer un 5-formylvalérate d'alkyle, dans lequel les étapes suivantes sont exécutées :

   (a) hydroformylation d'un 3- et/ou d'un 4-penténoate d'alkyle en présence d'un système catalyseur contenant du rhodium et un catalyseur au phosphite multidenté, par quel moyen le mélange de la réaction d'hydroformylation contient 1,05 à 1,2 mol de ligand phosphite multidenté par mol de rhodium,
   (b) séparer le système catalyseur du mélange réactionnel obtenu dans l'étape (a) aboutissant à une fraction contenant les composés aldéhydiques linéaires et ramifiés, le composé de départ non converti et les isomères à double liaison de celui-ci, et une fraction contenant le système catalyseur et les composés à haut point d'ébullition,
   (c) purger une partie de la fraction contenant le système catalyseur et les composés à haut point d'ébullition, et recycler le reste vers l'étape (a),
   (d) séparer le système catalyseur de la purge avec le procédé selon l'une quelconque des revendications 1 à 7,
   (e) ré-utiliser le système catalyseur obtenu dans l'étape (d) vers l'étape (a),
   (f) séparer la fraction contenant les composés aldéhydiques linéaires et ramifiés, le composé de départ non converti et les isomères à double liaison de celui-ci obtenus dans l'étape (b) en deux ou plus étapes de séparation dans une fraction contenant l'aldéhyde linéaire, une fraction contenant les composés aldéhydiques ramifiés, une fraction contenant le composé de départ non converti et les isomères à double liaison de celui-ci et une fraction contenant au moins le composé valérate d'alkyle,
   (g) recycler la fraction contenant le composé de départ non converti et les isomères à double liaison de celui-ci obtenus dans l'étape (f) vers l'étape (a),
   (h) alimenter au moins une partie de la fraction contenant au moins le composé valérate d'alkyle vers l'étape (d).

9. Procédé selon la revendication 1, pour séparer un complexe de ligand rhodium/phosphite bidenté et un ligand phosphite libre à partir d'un mélange d'hydroformylation contenant aussi des composés d'hydroformylation à haut point d'ébullition, en mettant en contact ledit mélange avec une membrane polymérique, dans lequel ledit mélange

d'hydroformylation est dérivé d'un procédé d'hydroformylation pour la préparation d'un 5-formylvalérate d'alkyle, en faisant réagir le 3-penténoate d'alkyle et/ou le 4-penténoate d'alkyle avec du monoxyde de carbone et de l'hydrogène, en utilisant un catalyseur au complexe de ligand rhodium/phosphite bidenté, et ledit mélange est dilué avec du valérate d'alkyle ou un mélange contenant du valérate d'alkyle et un ou plusieurs composés penténoate d'alkyle, jusqu'à ce que la quantité des composés à haut point d'ébullition dans ledit mélange soit ≤ 50 % du poids (par rapport à la quantité totale du mélange), et la quantité des composés avec un poids moléculaire compris entre x ± 200, soit ≤ 20 % du poids (par rapport à la quantité totale du mélange), par quel moyen x est la limite de la membrane.

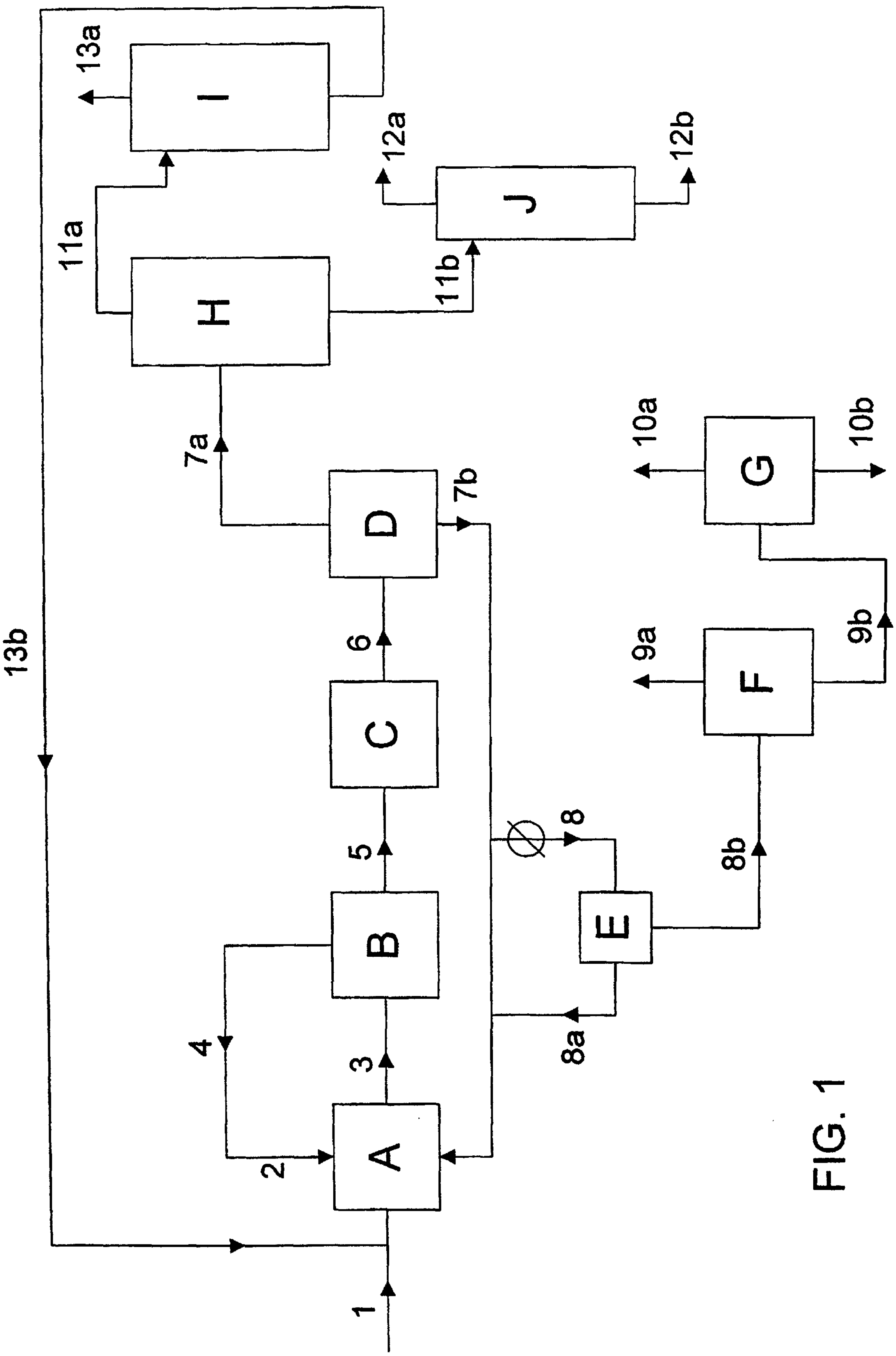
A
B
C
D
E
F
G
H
I
J
1
2
3
4
5
6
7a
7b
8
8a
8b
9a
9b
10a
10b
11a
11b
12a
12b
13a
13b

FIG. 1

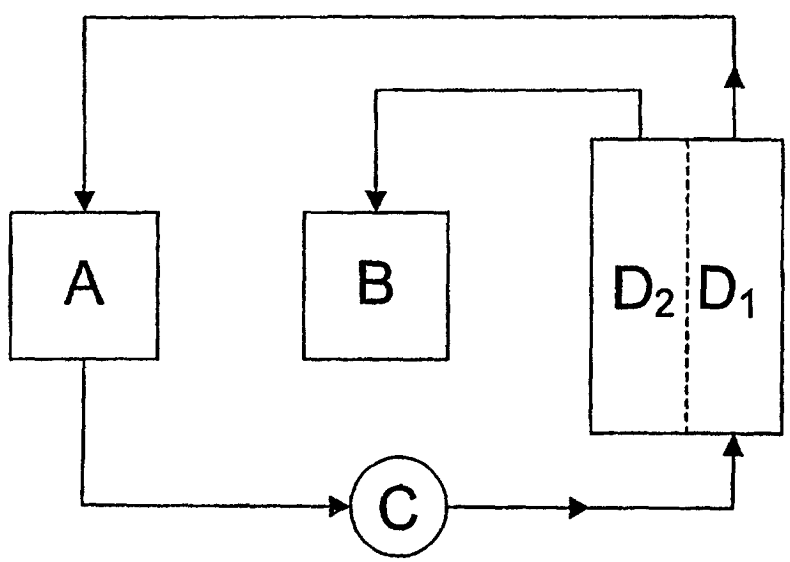


A = CONCENTRATE CONTAINER

b = PERMEATE CONTAINER

C = PUMP

$D_1$ = MEMBRANE MODULE CONCENTRATE SIDE

$D_2$ = MEMBRANE MODULE PERMEATE SIDE

FIG. 2